# FASCICULE DE BREVET EUROPEEN

(11) **EP 3 921 002 B1**
(45) Date de publication et mention de la délivrance du brevet: **20.11.2024**
(21) Numéro de dépôt: 20706793.5
(22) Date de dépôt: 03.02.2020
(51) Int. Cl.: A61M 15/00

(54) **DISPOSITIF DE DISTRIBUTION DE PRODUIT FLUIDE SYNCHRONISE AVEC L'INHALATION**
VORRICHTUNG ZUR INHALATIONSSYNCHRONISIERTEN ABGABE EINES FLÜSSIGEN PRODUKTS
DEVICE FOR THE INHALATION-SYNCHRONIZED DISPENSING OF A FLUID PRODUCT

(30) Priorité: 04.02.2019 FR 1901076
(43) Date de publication de la demande: 15.12.2021
(73) Titulaire: APTAR France SAS, 27110 Le Neubourg (FR)
(72) Inventeur: FABIEN, David, 29290 Saint Renan (FR); CAVATORTA, Matthieu, 78770 Autouillet (FR); TOURNOIS, Jeremy, 76800 Saint-Étienne-du-Rouvray (FR); WALTER, Matthieu, 78450 Villepreux (FR)
(74) Mandataire: CAPRI
(86) Numéro de dépôt international: PCT/FR2020/050170
(87) Numéro de publication internationale: WO 2020/161420

(56) Documents cités:
- WO-A1-2017/112451
- WO-A1-2017/178764
- WO-A1-2018/048795
- WO-A1-2019/170718
- US-A- 5 060 643

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité. Un autre inconvénient des dispositifs existants est que la valve est généralement sous contrainte avant l'inhalation de l'utilisateur, parfois même pendant les périodes de stockage entre deux actionnements, avec par conséquent des risques de fuites et de dysfonctionnements de la valve. Par ailleurs, dans les dispositifs existants, la valve reste en général en position actionnée après inhalation, jusqu'à ce que l'utilisateur ramène le dispositif en position de repos, par exemple en refermant le capot. Ici aussi il existe un risque de fuite dans la valve, avec par conséquent la dose suivante qui peut être incomplète et/ou une perte du produit fluide contenu dans le réservoir.

Les documents WO2017178764, WO2018048795, WO2017112451, US5060643, WO2004028608, US3456646, US5119806, NZ562769, US2008156321, WO2008070516, WO2010003846 et WO2013178951 décrivent des dispositifs de l'état de la technique.

Le document WO2019170718 décrit un dispositif de l'état de la technique selon l'article 54(3) CBE.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace et une précision de dosage à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui minimise les risques de fuite dans la valve, avant et/ou après inhalation.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente invention a également pour but de fournir un dispositif de distribution de produit fluide synchronisé avec l'inhalation qui évite les risques de dysfonctionnement de la valve dus à un mauvais remplissage de la chambre de valve après actionnement.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant par rapport audit corps, une valve doseuse, comportant une soupape déplaçable entre une position de repos et une position d'actionnement, étant assemblée sur ledit réservoir pour distribuer sélectivement le produit fluide à chaque actionnement, ledit dispositif comportant:
- un élément de blocage déplaçable et/ou déformable entre une position de blocage, dans laquelle ladite valve doseuse ne peut pas être actionnée, et une position d'actionnement, dans laquelle ladite valve doseuse peut être actionnée,
- un élément déclencheur déplaçable et/ou déformable entre une position de verrouillage, dans laquelle il bloque ledit élément de blocage dans sa position de blocage, et une position de libération, dans laquelle il ne bloque pas ledit élément de blocage,
- un système de déclenchement commandé par l'inhalation comportant un organe sensible à l'inhalation, déformable et/ou déplaçable sous l'effet de l'inhalation, ledit organe sensible à l'inhalation coopérant avec ledit élément déclencheur, de sorte que lorsque ledit organe sensible à l'inhalation se déforme et/ou se déplace, il déplace et/ou déforme ledit élément déclencheur vers sa position de libération, permettant ainsi le déplacement et/ou la déformation dudit élément de blocage de sa position de blocage vers sa position d'actionnement, et
- un organe d'actionnement, déplaçable entre une position de repos et une position d'actionnement sous l'effet d'un ressort, ledit organe d'actionnement coopérant avec ledit élément de blocage, de telle sorte qu'en position de blocage dudit élément de blocage, ledit élément de blocage empêche un déplacement axial dudit organe d'actionnement, et en position d'actionnement dudit élément de blocage, ledit élément de blocage permet un déplacement axial dudit organe d'actionnement, et un compteur de doses,
- ledit dispositif comportant un système de libération automatique de soupape, qui ramène automatiquement ladite soupape de ladite valve vers sa position de repos après actionnement de la valve, indépendamment de la position dudit organe d'actionnement.

Avantageusement, ledit dispositif comporte un capot déplaçable, notamment pivotant sur la corps, entre une position de fermeture de l'embout buccal et une position d'ouverture de l'embout buccal, ledit capot coopérant avec ledit organe d'actionnement de telle sorte qu'en position de fermeture, il bloque ledit organe d'actionnement contre un déplacement axial dans le corps, et lorsqu'il est ramené de sa position d'ouverture vers sa position de fermeture, il ramène l'organe d'actionnement vers sa position de repos en rechargeant ledit ressort.

Avantageusement, ledit système de libération automatique de soupape comporte deux leviers, un élément de poussée et un ressort de leviers, ledit élément de poussée étant destiné à venir en contact avec ledit réservoir, ledit élément de poussée étant relié audit organe de poussée, avec interposition dudit ressort de leviers.

Avantageusement, chaque levier est assemblé via des plots de manière pivotante dans une came dudit organe de poussée et coopère avec ledit élément de poussée pour transmettre la force exercée par ledit ressort audit élément de poussée.

Avantageusement, en position verrouillée, avant inhalation, lesdits leviers sont d'un côté en appui sur un épaulement dudit élément de poussée, et de l'autre côté, lesdits leviers sont en contact de doigts de verrouillage formés dans ledit couvercle et qui s'étendent axialement vers le bas à partir du fond dudit couvercle, lesdits doigts de verrouillage empêchant lesdits leviers de pivoter hors de contact avec ledit épaulement dudit élément de poussée.

Avantageusement, en position d'actionnement, après inhalation, lesdits leviers ne coopèrent plus avec lesdits doigts de verrouillage dudit couvercle, de sorte qu'ils peuvent alors pivoter dans ledit organe de poussée hors de contact avec ledit épaulement dudit élément de poussée, de sorte que ledit réservoir peut remonter vers sa position de repos sous l'effet du ressort de rappel de ladite valve, avec lesdits leviers passant à travers des trous dudit élément de poussée.

Avantageusement, lors du réarmement du dispositif, lorsque l'utilisateur referme ledit capot, ledit organe d'actionnement et ledit organe de poussée reviennent vers leurs positions de repos, en comprimant ledit ressort, et lesdits leviers se déplacent axialement avec ledit organe de poussée jusqu'à ce qu'ils entrent en contact avec lesdits doigts de verrouillage dudit couvercle, qui sollicitent lesdits plots à se déplacer radialement vers l'extérieur dans lesdits cames, ce qui permet auxdits leviers de revenir autour desdits doigts de verrouillage, ledit ressort de leviers ramenant alors lesdits leviers en appui sur ledit épaulement dudit élément de poussée.

Avantageusement, ledit élément de blocage est monté pivotant sur le corps autour d'un axe de pivotement B et ledit élément déclencheur est monté pivotant sur le corps autour d'un axe de pivotement C, lesdits axes B et C étant parallèles.

Avantageusement, ledit organe d'actionnement comporte une projection axiale coopérant avec ledit élément de blocage, de telle sorte qu'en position de blocage dudit élément de blocage, ladite projection axiale dudit organe d'actionnement coopère avec une extension de blocage axiale dudit élément de blocage pour ainsi empêcher un déplacement axial dudit organe d'actionnement, et en position d'actionnement dudit élément de blocage, ladite projection axiale dudit organe d'actionnement coopère avec un évidement axial dudit élément de blocage pour ainsi permettre un déplacement axial dudit organe d'actionnement.

Avantageusement, en position de blocage dudit élément de blocage, ladite projection axiale dudit organe d'actionnement sollicite ledit élément de blocage vers sa position d'actionnement.

Selon l'invention, ledit élément de blocage comporte une projection de verrouillage qui, en position de verrouillage de l'élément déclencheur, coopère avec un épaulement de verrouillage dudit élément déclencheur pour définir une serrure empêchant le déplacement et/ou la déformation dudit élément de blocage hors de sa position de blocage,

Avantageusement, ladite serrure forme, en position de verrouillage de l'élément déclencheur, un premier point de contact entre ledit élément de blocage et ledit élément déclencheur, ledit élément de blocage comportant une projection d'appui qui, en position de verrouillage de l'élément déclencheur, coopère avec une surface d'appui dudit élément déclencheur pour former, en position de verrouillage de l'élément déclencheur, un second point de contact entre ledit élément de blocage et ledit élément déclencheur, ledit second point de contact étant, en position de verrouillage de l'élément déclencheur, à une distance dudit axe C de l'élément déclencheur supérieure à la distance entre ledit axe C et ledit premier point de contact.

Avantageusement, ledit dispositif comporte :
- un couvercle fixé, notamment vissé ou encliqueté, sur ledit corps, et
- un organe de poussée coopérant avec ledit organe d'actionnement et monté coulissant axialement dans ledit couvercle, ledit ressort étant disposé entre un fond dudit couvercle et ledit organe de poussée,
dans lequel, avant inhalation, ledit organe de poussée est hors de contact dudit réservoir, de sorte que la force exercée sur ledit organe de poussée par ledit ressort n'est pas transmise audit réservoir, et lors de l'inhalation, ledit organe de poussée se déplace axialement avec ledit organe d'actionnement pour venir en contact avec ledit réservoir et déplacer ledit réservoir axialement dans ledit corps pour actionner ladite valve.

Avantageusement, le dispositif comporte un indicateur pour indiquer à l'utilisateur que la distribution de produit fluide a été réalisée.

Avantageusement, ledit indicateur est un indicateur visuel et/ou sonore.

Avantageusement, le dispositif comporte un compteur électronique.

Avantageusement, avant la première utilisation du dispositif, ledit compteur électronique est en mode veille, ledit compteur électronique comportant un contacteur, ledit organe d'actionnement, lors de son premier déplacement vers sa position d'actionnement, contactant ledit contacteur pour mettre ledit compteur électronique en mode de fonctionnement normal.

Avantageusement, ledit compteur électronique comporte au moins un accéléromètre.

Avantageusement, ledit organe sensible à l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de verrouillage vers sa position de libération.

Avantageusement, ledit embout buccal comporte une ouverture reliée avec l'intérieur du corps, ladite ouverture étant fermée en début d'inhalation par un clapet, de sorte que le flux d'inhalation est initialement principalement transmis au système de déclenchement par l'inhalation.

Avantageusement, ledit clapet est ouvert lorsque ledit organe d'actionnement se déplace axialement ensemble avec ledit réservoir.

Ces caractéristiques et avantages et d'autres apparaîtront plus clairement au cours de la description détaillée suivante, faite en référence aux dessins joints, donnés à titre d'exemples non limitatifs, et sur lesquels :
La figure 1 est une vue schématique en section transversale d'un dispositif de distribution de produit fluide selon un premier mode de réalisation avantageux, en position de repos,
La figure 2 est une vue similaire à celle de la figure 1, après ouverture du capot et avant inhalation,
La figure 3 est une vue similaire à celle de la figure 2, après inhalation,
La figure 4 est une vue similaire à celle de la figure 2, montrant une variante de réalisation,
La figure 5 est une vue similaire à celle de la figure 4, après inhalation,
La figure 6 est une vue schématique de face du dispositif de la figure 1, avant inhalation,
La figure 7 est une vue similaire à celle de la figure 6, après inhalation,
La figure 8 est une vue schématique en section d'une variante de réalisation du dispositif de la figure 1, avant assemblage,
La figure 9 est une vue similaire à celle de la figure 8, en cours d'assemblage,
La figure 10 est une vue schématique de détail d'une partie du dispositif de la figure 9, avant sertissage
La figure 11 est une vue similaire à celle de la figure 10, après sertissage,
La figure 12 est une vue schématique en perspective éclatée d'un dispositif de distribution de produit fluide selon un second mode de réalisation avantageux,
Les figures 13 à 15 sont des vues schématiques en section transversale du dispositif de la figure 12, respectivement en position de repos, après ouverture du capot et avant inhalation, et après inhalation,
Les figures 16 à 18 sont des vues schématiques agrandie de la serrure du dispositif des figures 13 à 15, respectivement avant inhalation, en début d'inhalation et en fin d'inhalation,
Les figures 19 à 22 sont des vues schématiques de détail du système de libération de soupape, pendant différentes étapes de son actionnement et réarmement,
La figure 23 est une vue schématique en perspective découpée de l'organe de poussée,
La figure 24 est une vue schématique en perspective de d'un levier du système de libération de soupape,
La figure 25 est une vue schématique en perspective de l'élément de poussée du système de libération de soupape,
La figure 26 est une vue schématique en perspective de l'organe d'actionnement,
La figure 27 est une vue schématique en perspective du capot,
La figure 28 est une vue schématique en perspective de l'élément déclencheur, et
La figure 29 est une vue schématique en perspective de l'élément de blocage.

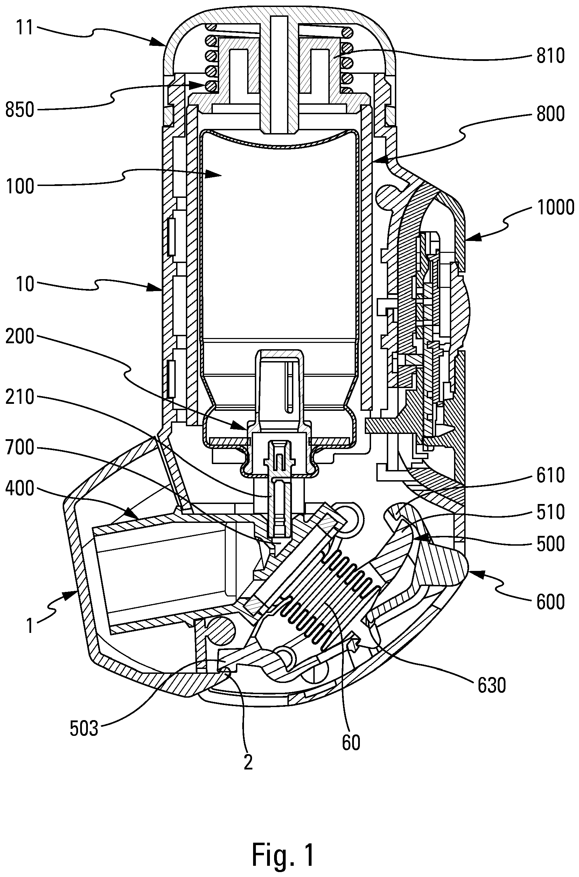

Dans la description, les termes "supérieur", "inférieur", "haut" et "bas" se réfèrent à la position du dispositif représentée notamment sur les figures 1 à 9 et 13 à 15. Les termes "axial" et "radial", sauf si autrement spécifié, se réfèrent à l'axe central vertical de la valve. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent divers modes de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps 10 pourvu d'un embout buccal 400.

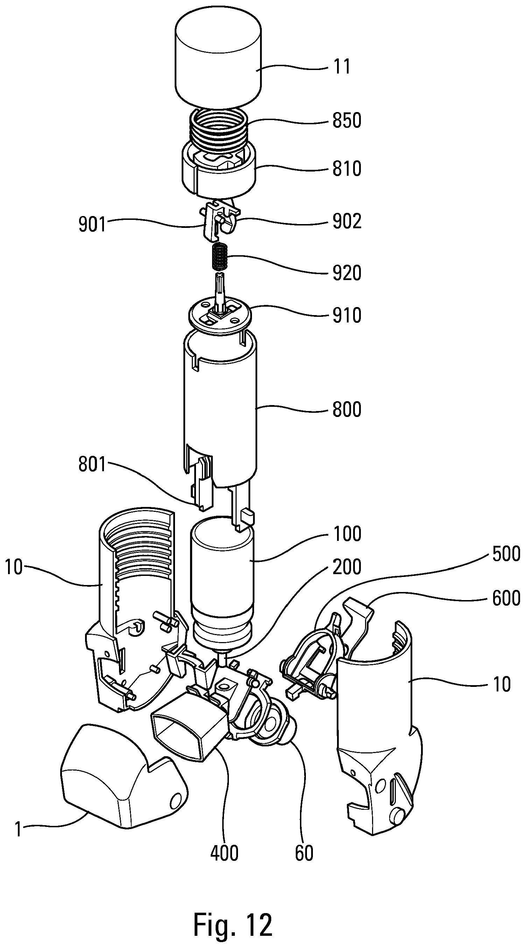

Le corps 10 peut être réalisé en une seule pièce ou en plusieurs pièces assemblées les unes aux autres. La figure 12 montre un exemple dans lequel le corps 10 est formé de deux demi-coques, mais d'autres mises en oeuvre sont possibles. Dans la suite de la description, le corps sera désigné de manière globale par la référence numérique 10.

L'embout buccal 400 définit un orifice de distribution à travers lequel l'utilisateur va inhaler lors de l'utilisation du dispositif. L'embout buccal 400 peut être réalisé d'une pièce avec le corps 10. Dans les exemples représentés sur les dessins, il est assemblé sur une partie inférieure du corps 10.

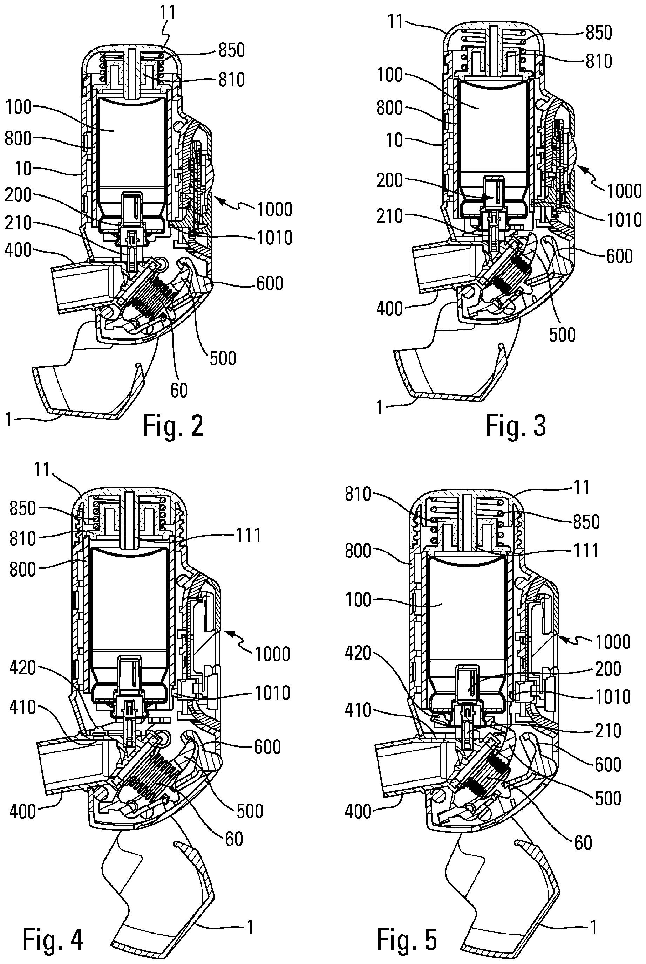

Un capot de protection 1 amovible est prévu pour couvrir ledit embout buccal 400, notamment lors des périodes de stockage. Ce capot 1 est déplaçable, de préférence en étant monté pivotant sur le corps 10, entre une position de fermeture, visible sur les figures 1, 8, 9 et 13, et une position d'ouverture, visible sur les figures 2 à 7, 14 et 15.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve et une soupape 210 axialement déplaçable lors de l'actionnement par rapport audit corps de valve, et donc par rapport audit réservoir 100, entre une position de repos et une position d'actionnement. Cette valve doseuse 200 peut être d'un type quelconque approprié. Elle est fixée au réservoir 100 par un élément de fixation approprié, de préférence une capsule sertie, de préférence avec interposition d'un joint de col.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale, qui est la position d'actionnement.

L'orifice de sortie de la soupape 210 de ladite valve doseuse 200 est relié via un canal audit embout buccal 400, à travers lequel l'utilisateur inhale le produit distribué. De manière connue, ladite soupape 210 est reçue dans un puits de soupape 700 qui définit au moins partiellement ledit canal.

Un organe d'actionnement 800 est avantageusement assemblé autour du réservoir 100. Cet organe d'actionnement 800 comporte un manchon creux disposé dans le corps 10 autour du réservoir 100. Un organe de poussée 810 est monté sur le bord axial distal dudit organe d'actionnement 800, ledit organe de poussée 810 étant reçu dans un élément de couvercle 11 fixé sur le bord axial supérieur dudit corps 10. Un ressort 850 est disposé entre un fond dudit élément de couvercle 11 et ledit organe de poussée 810. En position de repos, et jusqu'à l'inhalation, le ressort 850 est précontraint, et exerce donc une force axiale F sur l'organe de poussée 810 qui transmet cette force à l'organe d'actionnement 800. L'organe d'actionnement 800 est déplaçable axialement, notamment coulissant, par rapport audit corps 10 entre une position de repos et une position d'actionnement. Un bord inférieur 802 de l'organe d'actionnement 800 coopère avec le capot 1 de telle sorte qu'en position de fermeture du capot 1, ledit organe d'actionnement 800 est bloqué en position de repos, le bord inférieur 802 étant en butée sur une partie 3 dudit capot 1. De plus, ledit capot 1 comporte une came 4 qui coopère avec ledit bord inférieur 802 lorsque le capot 1 est ramené de sa position ouverte vers sa position fermée, pour ramener ledit organe d'actionnement 800 de sa position d'actionnement vers sa position de repos. Lorsque l'organe d'actionnement 800 revient vers sa position de repos, il ramène également l'organe de poussée 810, ce qui provoque la compression du ressort 850. Le ressort 850 est donc rechargé après chaque actionnement lorsque l'utilisateur referme le capot 1.

Le dispositif comporte un élément de blocage 500 déplaçable et/ou déformable entre une position de blocage, dans laquelle ladite valve doseuse 200 ne peut pas être actionnée, et une position d'actionnement, dans laquelle ladite valve doseuse 200 peut être actionnée.

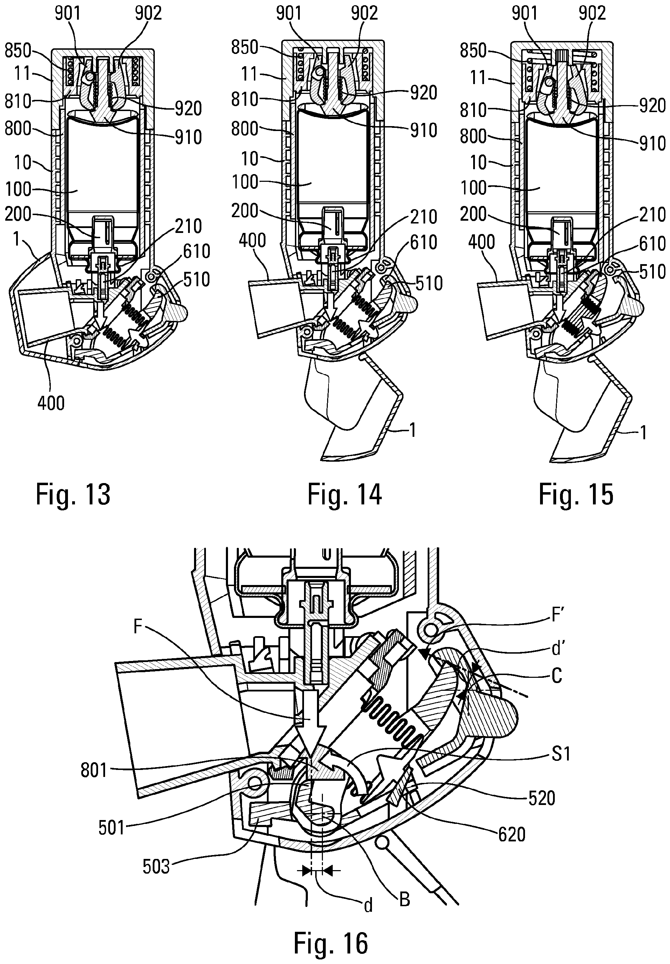

L'élément de blocage 500 est avantageusement monté pivotant sur le corps 10 autour d'un axe B (mieux visible sur les figures 16 à 18) entre la position de blocage et la position d'actionnement.

Avant inhalation, ledit élément de blocage 500 est en position de blocage, et c'est l'inhalation de l'utilisateur à travers l'embout buccal 400 qui déplace et/ou déforme ledit élément de blocage 500 vers sa position d'actionnement. En d'autres termes, tant que l'utilisateur n'a pas inhalé, l'actionnement de la valve doseuse 200 est impossible, et ce n'est que lorsqu'il inhale que ladite valve doseuse 200 peut être actionnée, par déplacement axial du réservoir 100 dans le corps 10.

Comme cela sera décrit plus en détails ci-après, l'élément de blocage 500, en position de blocage, empêche le déplacement axial de l'organe d'actionnement 800 dans le corps 10. Lors de l'inhalation, cet élément de blocage 500 est déplacé et/ou déformé de telle sorte qu'il ne bloque plus le déplacement axial de l'organe d'actionnement 800 dans le corps 10. Ainsi, après inhalation, un tel déplacement axial de l'organe d'actionnement 800 provoque le déplacement axial du réservoir 100 et donc l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation.

En position de fermeture du capot 1, une partie de blocage 2 du capot 1 coopère avec une partie saillante 503 de l'organe de blocage 500 pour bloquer celui-ci en position de blocage, comme visible notamment sur la figure 1. Ce blocage est supprimé lors de l'ouverture du capot 1.

L'ouverture du capot 1 libère donc deux blocages fournis par le capot en position fermée : d'une part le blocage en déplacement axial de l'organe d'actionnement 800 et d'autre part le blocage en pivotement de l'organe de blocage 500.

Le dispositif comporte un système de déclenchement commandé par l'inhalation de l'utilisateur, qui est destiné à déplacer et/ou déformer ledit élément de blocage 500 de sa position de blocage vers sa position d'actionnement, lorsque l'utilisateur inhale à travers l'embout buccal 400.

Ce système de déclenchement comporte un organe sensible à l'inhalation 60 déformable et/ou déplaçable sous l'effet de l'inhalation, cet organe sensible à l'inhalation 60 étant adapté, lorsqu'il se déforme et/ou se déplace, à permettre le déplacement et/ou la déformation dudit élément de blocage 500 de sa position de blocage vers sa position d'actionnement.

Comme cela sera décrit plus en détails ci-après, l'organe sensible à l'inhalation peut être réalisé sous la forme d'une chambre d'air déformable 60, par exemple un soufflet ou une poche déformable.

De cette manière, le système de déclenchement commandé par l'inhalation n'est pas situé dans le flux de l'aspiration de l'utilisateur mais est formé par une chambre spécifique, à savoir la chambre d'air 60. Ceci diffère des systèmes fonctionnant à l'aide d'un volet qui se déplace/déforme dans le flux d'aspiration, dans lesquels, après déclenchement, l'utilisateur va aspirer l'air qui se trouve de chaque côté du volet. Ici, le système fonctionne en dépression et l'utilisateur n'aspire que le petit volume d'air qui était à l'intérieur de la chambre d'air 60 avant sa déformation. Le système selon l'invention est ainsi beaucoup plus stable et performant.

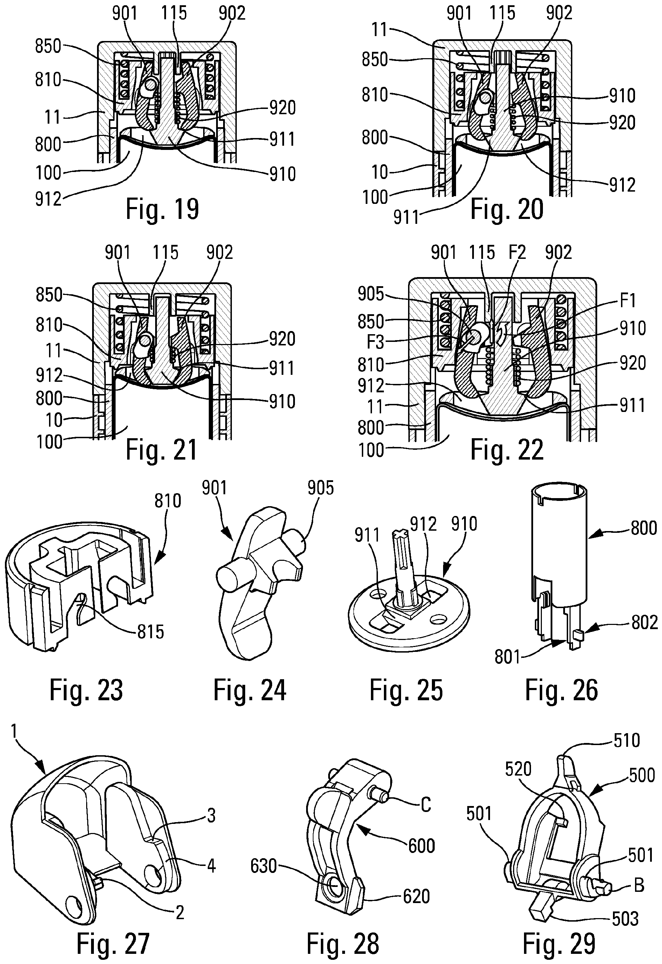

L'élément de blocage 500 comporte au moins une, de préférence deux extensions de blocage 501, qui coopère chacune en position de blocage avec une projection axiale 801 de l'organe d'actionnement 800. La figure 29 représente une vue en perspective de cet élément de blocage 500.

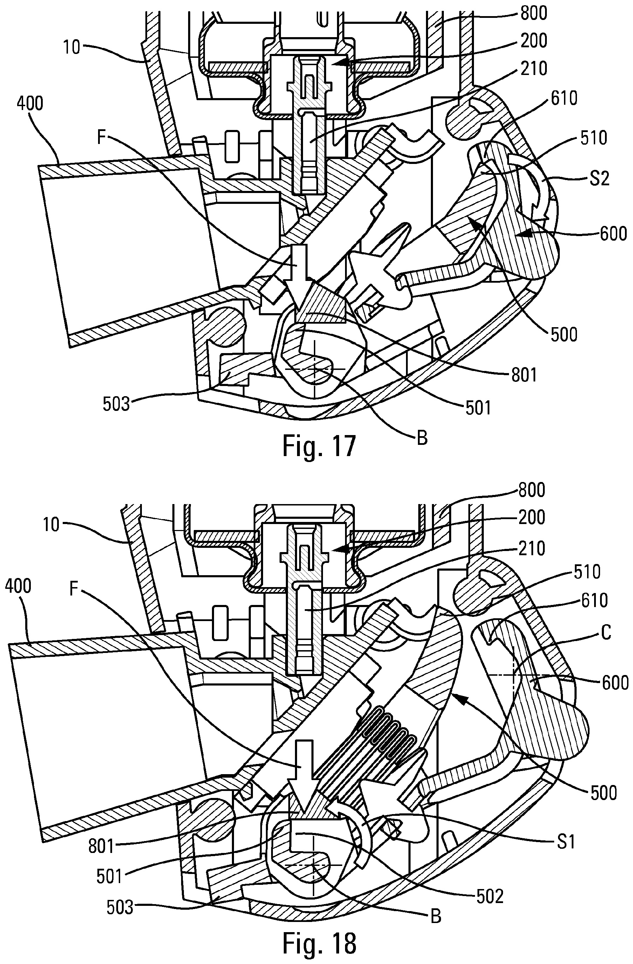

Lorsque l'élément de blocage 500 se déplace vers sa position d'actionnement, notamment par pivotement autour de l'axe B, chaque extension de blocage 501 se déplace hors de contact avec la projection axiale 801 respective. En particulier, ledit élément de blocage 500 comporte de manière adjacente à chaque extension de blocage 501 un évidement axial 502, visible sur la figure 18, dans lequel la projection axiale 801 respective peut coulisser axialement, pour ainsi permettre un coulissement axial dudit organe d'actionnement 800 dans ledit corps 10, provoquant le déplacement axial du réservoir 100 et l'actionnement de la valve 200 avec la distribution d'une dose de produit fluide.

L'élément de blocage 500 est retenu en position de blocage par un élément déclencheur 600. La figure 28 représente une vue en perspective de cet élément déclencheur 600. Cet élément déclencheur 600 est avantageusement monté pivotant sur le corps 10 autour d'un axe C (visible sur les figures 16 et 18), entre une position de verrouillage, dans laquelle il bloque ledit élément de blocage 500 dans sa position de blocage, et une position de libération, dans laquelle il ne bloque plus ledit élément de blocage 500.

Avantageusement, les axes B et C sont parallèles.

L'élément de blocage 500 et l'élément déclencheur 600 définissent ensemble une serrure. En particulier, ledit élément déclencheur 600 comporte un épaulement de verrouillage 610 qui, en position de verrouillage, coopère avec une projection de verrouillage 510 de l'élément de blocage 500, empêchant le pivotement dudit élément de blocage 500 hors de sa position de blocage. Ainsi, lorsque ledit élément déclencheur 600 est en position de verrouillage, il empêche le déplacement de l'élément de blocage 500 vers sa position d'actionnement, ce qui bloque le déplacement axial du réservoir 100 et donc l'actionnement de la valve doseuse 200.

Le système de blocage de la présente invention comporte donc deux étages : un premier étage formé par la serrure entre l'élément de blocage 500 et l'élément déclencheur 600, et un second étage formé par le blocage entre l'élément de blocage 500 et l'organe d'actionnement 800.

Ce système de blocage permet le déverrouillage d'un gros effort (typiquement environ 40-45N) par un petit effort généré par l'inhalation. L'élément de blocage 500 arrête la translation de l'organe d'actionnement 800 lorsqu'il est soumis à un effort F (de 45N par exemple) par l'appui du ressort 850 sur l'organe d'actionnement 800 via l'organe de poussée 810. Cet élément de blocage 500 interagit avec l'élément déclencheur 600 et est bloqué/libéré par celui-ci. Le déplacement dudit élément déclencheur 600 est commandé par l'inhalation.

La géométrie du système de blocage permet une amplification (effort déverrouillé/effort déverrouillant) très importante, typiquement de l'ordre de 100.

L'élément de blocage 500 et l'élément déclencheur 600 ont de préférence deux points de contact géométriquement distant:
- un premier point de contact, formé par la serrure définie entre l'épaulement de verrouillage 610 et la projection 510, se trouvant avantageusement proche de l'axe de pivotement C de l'élément déclencheur 600;
- un second point de contact éloigné du premier point de contact, formé par la coopération d'une projection latérale 520 de l'élément de blocage 500 avec une surface d'appui 620 de l'élément déclencheur 600; avantageusement, ce second point de contact est, en position de verrouillage, à une distance de l'axe C de l'élément déclencheur 600 supérieure à la distance entre ledit axe C et le premier point de contact; avantageusement, ce second point de contact est le premier contact qui se rompt lors de l'actionnement du dispositif, dès que l'utilisateur commence à inhaler.

En position de blocage, après ouverture du capot 1 et avant inhalation, la force axiale F générée par le ressort 850 sur l'organe d'actionnement 800 est appliquée par les projections axiales 801 de l'organe d'actionnement 800 sur l'élément de blocage 500 au niveau des extensions 501, avec pour effet de solliciter ledit élément de blocage 500 en rotation dans un premier sens S1, visible sur la figure 16, qui renforce la position fermée de la serrure et la rend stable.

La force de déverrouillage générée par l'inhalation est appliquée sur l'élément déclencheur 600 par l'organe sensible à l'inhalation 60, de préférence en un point 630 éloigné de l'axe du pivot C. Cette force de déverrouillage vise à faire tourner ledit élément déclencheur 600 dans le sens S2 contraire à S1, comme illustré sur la figure 17.

Le couple que subit l'élément de blocage 500 est maitrisé par la distance d entre l'axe d'effort sur lequel la force F est appliquée sur les extensions de blocage 501 de l'élément de blocage et l'axe de pivot B dudit élément de blocage 500. On cherche à avoir une distance d la plus faible possible afin d'avoir le couple le plus faible possible. Cette distance d, visible sur la figure 16, est non nulle, et est inférieure à 2 mm, avantageusement inférieure à 1 mm.

Le couple que subit l'élément déclencheur 600 est maitrisé par la distance d' entre l'axe d'effort portant la force F' que subit l'élément déclencheur 600 de la part de l'élément de blocage 500 et l'axe de pivot C dudit élément déclencheur 600. Ici aussi, on cherche à avoir une distance d' la plus faible possible afin d'avoir le couple le plus faible possible. Cette distance d', visible sur la figure 16, est non nulle, et est inférieure à 2 mm, avantageusement inférieure à 1 mm.

Grâce à ce système d'effort de la serrure, l'effort nécessaire pour faire pivoter l'élément déclencheur 600 est très faible et peut être généré par l'organe sensible à l'inhalation 60, qui permet de transformer la dépression générée par l'inhalation en force de déverrouillage.

Avantageusement, comme représenté dans la variante des figures 4 et 5, l'embout buccal 400 comporte une ou plusieurs ouverture(s) 410 reliée(s) avec l'intérieur du corps 10. Cette au moins une ouverture 410 est fermée au repos et en début d'inhalation par un clapet 420, de sorte que le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément de blocage 500 se déplace vers sa position d'actionnement sous l'effet de l'inhalation, et donc le réservoir 100 se déplace axialement par rapport au corps 10 pour actionner la valve doseuse 200 pour distribuer une dose de produit fluide, ledit organe d'actionnement 800, ou en variante ledit réservoir 100, déplace ledit clapet 420 vers une position ouverte. Lorsque ladite au moins une ouverture 410 est ainsi ouverte, en cours d'actionnement, un appel d'air est généré, ce qui permet d'augmenter le flux d'inhalation. Ceci optimise la synchronisation entre la distribution de la dose et l'inhalation de l'utilisateur, et favorise une bonne distribution de la dose dans les poumons de l'utilisateur.

Avantageusement, l'élément déclencheur 600 peut être accessible de l'extérieur du corps 10. Ceci permet en cas de besoin de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité. Ceci permet aussi de réaliser un amorçage de la valve, si celle-ci est une valve classique nécessitant un tel amorçage.

Dans les modes de réalisation représentés sur les figures, l'organe sensible à l'inhalation 60 est réalisé sous la forme d'une chambre d'air déformable. Avantageusement, cette chambre d'air comprend une membrane déformable qui est reliée d'une part audit corps 10 et d'autre part audit élément déclencheur 600. Avantageusement, comme visible sur les figures, la membrane a une forme de soufflet et forme une chambre sensiblement étanche. D'autres formes sont possibles, notamment une simple poche ou diaphragme. Un plot peut fixer ladite membrane à un orifice ou bord 630 dudit élément déclencheur 600.

Lors de l'inhalation, la membrane déformable se déforme et/ou se contracte sous l'effet de la dépression générée par l'inhalation, provoquant le déplacement de l'élément déclencheur 600 de sa position de verrouillage vers sa position de libération. Ceci permet d'ouvrir la serrure définie entre l'élément de blocage 500 et l'élément déclencheur 600, et donc le déplacement dudit élément de blocage 500 de sa position de blocage vers sa position d'actionnement.

La valve 200 n'est donc actionnée qu'au moment de l'inhalation, de sorte que la dose de produit fluide est expulsée hors de l'orifice de distribution simultanément à l'inhalation.

En position de repos, avec la capot 1 fermé, l'organe de poussée 810 n'est pas en contact avec le réservoir 100, comme visible sur la figure 1. Ainsi, dans cette position, la force du ressort 850 est appliquée par l'organe de poussée 810 sur l'organe d'actionnement 800, qui la transmet au capot 1. Ainsi, pendant le stockage du dispositif, aucune contrainte n'est appliquée sur la valve 200, ce qui limite voire élimine les risques de fuites et/ou de dysfonctionnement de ladite valve.

Lorsque l'utilisateur souhaite utiliser le dispositif, il ouvre le capot 1. Ce faisant, il supprime le blocage axial de l'organe d'actionnement 800 par le capot 1 ainsi que le blocage en pivotement de l'élément de blocage 500 par le capot 1. Dans cette position, l'organe d'actionnement 800 est bloqué et empêché de coulisser axialement dans le corps 10 par les extensions de blocage 501 de l'élément de blocage 500 qui bloquent axialement les projections axiales 801 de l'organe d'actionnement 800. Comme visible sur la figure 2, dans cette position armée (capot ouvert, avant inhalation), l'organe de poussée 810 n'est toujours pas en contact avec le réservoir 100. Ainsi, dans cette position aussi, la force du ressort 850 est appliquée par l'organe de poussée 810 sur l'organe d'actionnement 800, qui la transmet à l'élément de blocage 500 qui la transmet à l'élément déclencheur 600. Ainsi, avant l'inhalation, aucune contrainte n'est appliquée sur la valve 200, ce qui limite voire élimine les risques de fuites et/ou de dysfonctionnement de ladite valve.

Lorsque l'utilisateur inhale à travers l'embout buccal 400, il va déformer la membrane déformable de l'organe sensible à l'inhalation 60, ce qui va faire pivoter l'élément déclencheur 600 fixé à ladite membrane déformable. Ce déplacement de l'élément déclencheur 600 va libérer la serrure formée entre l'épaulement de verrouillage 610 de l'élément déclencheur 600 et la projection 510 de l'élément de blocage 500. Sous l'effet de la force axiale F transmise par l'organe d'actionnement 800, l'élément de blocage 500 va pivoter permettant le coulissement axial dudit organe d'actionnement 800. De ce fait, l'organe de poussée 810, solidaire dudit organe d'actionnement 800, vient au contact du réservoir 100, provoquant ainsi le déplacement axial dudit réservoir 100 dans le corps 10 vers sa position de distribution, et donc l'actionnement de la valve 200.

Parallèlement, dans la variante des figures 4 et 5, l'organe d'actionnement 800 (ou en variante le réservoir 100) va ouvrir le clapet 420.

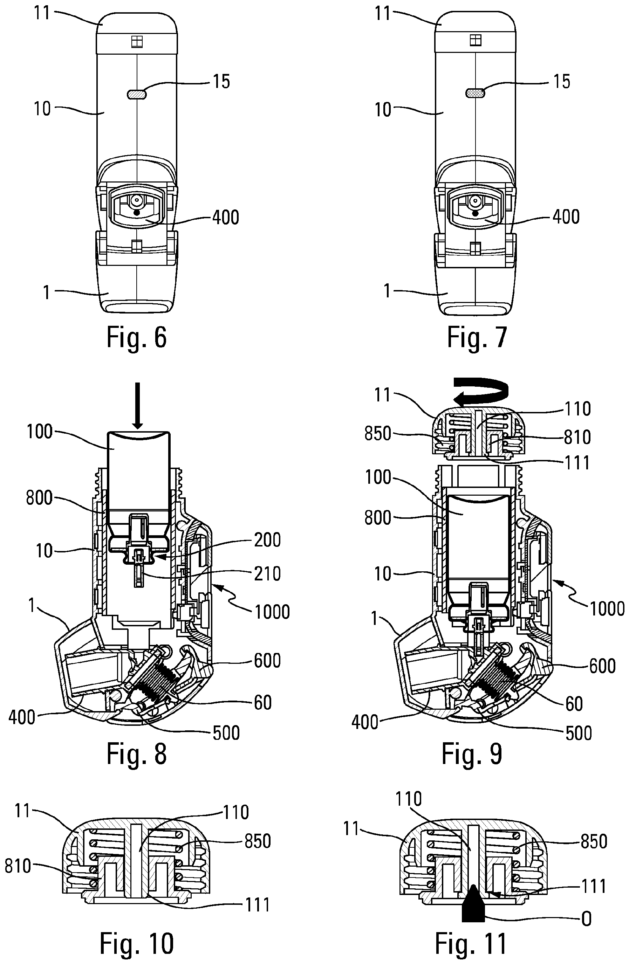

En fin d'inhalation, le dispositif comporte avantageusement des moyens de signalisation pour signaler à l'utilisateur qu'il doit refermer le capot 1. Ces moyens de signalisation peuvent comporter un indicateur visuel, comme illustré sur les figures 6 et 7. Dans cet exemple, une fenêtre 15 est formée dans le corps 10, à travers laquelle une partie colorée de l'organe d'actionnement 800 est visible de l'extérieur, formant moyens d'indication. Ainsi, en position de repos ou en position armée, avec la capot 1 ouvert mais avant inhalation, la fenêtre 15 peut indiquer une couleur claire, par exemple du vert, alors qu'après inhalation, c'est une couleur foncée, par exemple rouge qui peut s'afficher dans la fenêtre 15. Bien entendu, tout autre mise en oeuvre similaire est possible.

En variante, on pourrait avoir plusieurs fenêtres 15. La fenêtre (ou les fenêtres) 15 peut être positionnée différemment sur le dispositif, par exemple dans la partie supérieure du corps 10 ou sur le couvercle 11. Les moyens d'indication qui s'affichent dans la fenêtre 15 peuvent en variante comprendre des symboles, des chiffres, des lettres ou tout autre indication utile pour avertir l'utilisateur. Ces moyens d'indication peuvent être formés, par exemple formés par tampographie, directement sur l'organe d'actionnement 800, ou être formés sur une pièce fixée à celui-ci. Les moyens d'indication peuvent être réalisés sur toute autre pièce mobile lors de l'actionnement, par exemple l'organe de poussée 810, l'élément de blocage 500, l'élément déclencheur 600, l'organe sensible à l'inhalation 60.

Dans une autre variante, les moyens de signalisation peuvent comporter un indicateur sonore, tel qu'un haut-parleur, qui émet un son audible par l'utilisateur pour lui indiquer que le capot 1 doit être refermé.

Lorsque l'utilisateur referme le capot 1, l'organe d'actionnement 800 est repoussé axialement par ledit capot 1 vers sa position de repos, de sorte que ledit réservoir 100 peut remonter axialement dans le corps 10 en direction de sa position de repos sous l'effet du ressort de rappel de la valve 200, et simultanément la soupape 210 de la valve doseuse revient en position de repos, en chargeant une nouvelle dose de produit fluide dans la chambre de valve. L'élément déclencheur 600 est ramené dans sa position initiale, notamment par l'élasticité de la membrane. L'élément de blocage 500 revient dans sa position de blocage.

Le dispositif est alors prêt pour une autre utilisation.

Dans une variante de réalisation avantageuse, représentée sur les figures 8 à 11, l'assemblage final du dispositif est simplifié pour le laboratoire pharmaceutique qui commercialise le dispositif. En effet, dans la plupart des dispositifs existants, le réservoir rempli de principe actif est inséré dans le corps et ensuite il faut positionner plusieurs pièces au-dessus du réservoir, ce qui ne facilite pas les cadences de production et la simplicité d'assemblage. Pour éliminer cet inconvénient, la présente invention prévoit avantageusement de pré-assembler le sous-ensemble formé de l'organe de poussée 810, du ressort 850 et du couvercle 11. Ce sous-ensemble est précontraint lors de l'assemblage de ces trois pièces, par exemple en venant avec un outil chaud O déformer thermiquement le bord axial inférieur 111 d'un manchon axial central 110 du couvercle 11, autour duquel sont assemblés l'organe de poussée 810 et le ressort 850. Ledit bord axial inférieur 111 est ainsi déformé pour produire une collerette de rétention qui retient l'organe de poussée 810 et le ressort 850 dans le couvercle 11. Ce sous-ensemble ainsi formé peut alors être assemblé sur le corps 10 en une seule opération d'assemblage, par exemple par vissage ou par encliquetage. Il en résulte alors seulement deux opérations sur la ligne d'assemblage du laboratoire pharmaceutique : l'insertion du réservoir 100 dans le corps 10 (figure 8) et l'assemblage dudit sous-ensemble sur ledit corps 10 (figure 9). Lors de cet assemblage, l'organe d'actionnement 800 disposé dans le corps 10 va coopérer avec l'organe de poussée 810, pour le pousser axialement dans le couvercle 11 et ainsi charger le ressort 850. En version vissable, ce système peut aussi permettre de changer le réservoir. En effet, le dispositif pourrait être réutilisable et rechargeable dans un soucis d'écologie, notamment pour éviter de jeter trop de composants en plastique et/ou trop de composants électroniques.

Dans un mode de réalisation avantageux, représenté sur les figures 12 à 15 et 19 à 22, le dispositif comporte un système de libération automatique de soupape, qui ramène automatiquement la soupape 210 de la valve 200 vers sa position de repos après actionnement de la valve, indépendamment de la position du capot 1 et de la position de l'organe d'actionnement 800. Ainsi, dans cette variante, il n'y a pas de risque de dysfonctionnement, tel qu'un sous-dosage de la dose suivante, même si l'utilisateur tarde à refermer le capot.

Le système de libération de soupape comporte les pièces supplémentaires suivantes :
- deux leviers 901, 902,
- un élément de poussée 910,
- un ressort de leviers 920.

L'élément de poussée 910 est destiné à venir en contact du réservoir 100, et est relié à l'organe de poussée 810, avec interposition du ressort de leviers 920. Avantageusement, avant inhalation, l'élément de poussée 910 est très légèrement décalé axialement du réservoir 100, de sorte que dans cette position, il ne transmet aucune contrainte audit réservoir 100 ou à la valve 200. Ce n'est que lorsque l'utilisateur inhale, et libère le déplacement axial de l'organe d'actionnement 800, que l'élément de poussée 910 va venir au contact du réservoir 100.

Chaque levier 901, 902 est assemblé via des plots 905 de manière pivotante dans une came 815 de l'organe de poussée 810 et coopère avec ledit élément de poussée 910.

Lorsque l'utilisateur inhale, l'organe d'actionnement 800 et donc l'organe de poussée 810 se déplacent axialement vers le bas sous l'effet du ressort 850. Cette force est transmise par les leviers 901, 902 à l'élément de poussée 910, qui la transmet au réservoir 100, provoquant ainsi le déplacement axial dudit réservoir 100 et l'actionnement de la valve 200.

Le système de libération de soupape a donc pour but de libérer la transmission de la force du ressort 850 au réservoir 100 après l'inhalation, pour permettre audit réservoir 100 de revenir vers sa position de repos indépendamment de l'organe d'actionnement 800. Ceci permet de charger la prochaine dose dans la valve 200 immédiatement après inhalation, quand le dispositif est encore dans la position adéquate pour ce chargement, sans risque de mauvais dosage, par exemple en cas d'oubli de refermer le capot 1.

Le fonctionnement de ce système de libération de soupape est le suivant :
En position verrouillée, avant inhalation, visible sur la figure 19, les leviers 901 et 902 sont en appui sur un épaulement 911 de l'élément de poussée 910. Du côté opposé, les leviers sont en contact de doigts de verrouillage 115 formés dans le couvercle 11 et qui s'étendent axialement vers le bas à partir du fond dudit couvercle. Ces doigts de verrouillage 115 empêchent les leviers 901, 902 de pivoter hors de contact avec l'épaulement 911 de l'élément de poussée. Dans cette position, la force du ressort 850 est donc transmise par les leviers 901, 902 à l'élément de poussée 910 et donc au réservoir 100.

Lorsque l'organe d'actionnement 800 arrive en position d'actionnement, avec la valve 200 qui a été actionnée pour distribuer une dose, l'organe de poussée 810, l'élément de poussée 910 et les leviers 901, 902 ont coulissé axialement vers le bas par rapport au couvercle 11, comme visible sur la figure 20. Dans cette position, les leviers 901, 902 ne coopèrent plus avec les doigts de verrouillage 115 du couvercle 11. Ils peuvent alors pivoter dans l'organe de poussée 810 jusqu'à ce qu'ils ne soient plus en contact avec l'épaulement 911 de l'élément de poussée. Avantageusement, la libération de l'élément de poussée 910 se produit après expulsion de la dose par la valve, mais avant la fin de la course d'actionnement de celle-ci.

Lorsque les leviers 901, 902 ne sont plus en contact avec l'épaulement 911 de l'élément de poussée, chacun se trouve face à une ouverture 912 dudit élément de poussée 910, de sorte que le réservoir 100 peut remonter vers sa position de repos sous l'effet du ressort de rappel de la valve 200, avec les leviers 901, 902 qui passent à travers lesdits trous 912, comme visible sur la figure 21. Lors de ce retour du réservoir 100 vers sa position de repos, l'élément de poussée 910 coulisse axialement vers le haut ensemble avec le réservoir 100, par rapport à l'organe de poussée 810 et aux leviers 901, 902. Lors de ce déplacement, le ressort de leviers 920, dont la raideur doit être inférieure à celle du ressort de la valve 200, se comprime.

Lors du réarmement du dispositif, représenté sur la figure 22, lorsque l'utilisateur referme le capot 1, l'organe d'actionnement 800 et l'organe de poussée 810 reviennent vers leurs positions de repos, en comprimant le ressort 850. Lors de ce déplacement, les leviers 901, 902 subissent une force exercée par le ressort de leviers 920, qui les sollicitent en rotation, comme indiqué par la flèche F1. Tant que les leviers 901, 902 sont dans les trous 912 de l'élément de poussée 910, ils ne peuvent pas pivoter et se déplacent axialement avec l'organe de poussée 810, comme indiqué par la flèche F2. Lorsqu'ils entrent en contact avec les doigts de verrouillage 115 du couvercle 11, les plots 905 sont forcés à se déplacer radialement vers l'extérieur dans les cames 815, comme indiqué par la flèche F3, ce qui permet aux leviers 901, 902 de revenir autour desdits doigts de verrouillage 115 du couvercle 11. Lorsque l'organe d'actionnement 800 et l'organe de poussée 810 arrivent à leurs positions de repos, les leviers 901, 902 sont ressortis des trous 912 de l'élément de poussée, et le ressort de leviers 920 ramène lesdits leviers en appui sur l'épaulement 911.

Pour garantir un fonctionnement fiable du dispositif, le système de libération de soupape doit être actionné seulement après une course suffisante pour garantir que la distribution de la dose se produit. En raison des tolérances de fabrication des pièces, il peut être avantageux de prévoir un élément tampon entre l'élément de poussée 910 et le réservoir 100. Cet élément tampon, qui doit ralentir ou décaler l'actionnement du système de libération de soupape, peut être un élément élastique, tel qu'un ressort ou une pièce compressible, par exemple en élastomère. Sa résistance doit être d'une part supérieure à la force du ressort de la valve 200 en position d'actionnement de la soupape 210, de sorte que c'est d'abord la valve 200 qui est actionnée avant que l'élément tampon ne se déforme, et d'autre part inférieure à la force du ressort 850 en position d'actionnement de l'organe d'actionnement 800, pour garantir que l'élément tampon se comprimera en fin de course d'actionnement. En variante, on pourrait aussi utiliser un élément tampon formé par un vérin ou par une chambre à volume variable, remplie d'air ou d'un fluide, et pourvue d'un orifice de fuite.

Dans un mode de réalisation avantageux, le dispositif peut comporter un compteur de doses 1000, représenté sur les figures 1 à 9, qui peut être mécanique ou électronique, avantageusement assemblé dans le corps 10. Un tel compteur pourrait aussi être associé aux modes de réalisation des figures 12 à 22. Ce compteur 1000 peut notamment détecter les déplacements de l'organe d'actionnement 800 ou du réservoir 100. En variante, le compteur pourrait être relié à un capteur, notamment un capteur à membrane, qui détecte la distribution de la dose de produit fluide, par exemple dans le puits de soupape. D'autres moyens d'actionner un tel compteur sont aussi possibles, par exemple la détection du déplacement de la soupape 210 de la valve doseuse 200 par rapport au corps de valve.

Lorsque le compteur est électronique, il doit pouvoir avoir assez d'énergie électrique pour fonctionner durant toute la durée de stockage jusqu'à sa première utilisation. La batterie doit ensuite avoir une capacité suffisante, par exemple pour communiquer avec l'utilisateur (visualisation du nombre de dose restantes) et/ou avec une application tierce, et ce tout au long de son utilisation et au moins jusqu'à la date de péremption du médicament. Pour éviter une batterie trop grande, il faut réduire la consommation de la carte électronique avant la première utilisation. Pour se faire, l'électronique est avantageusement plongée dans un mode de veille, peu consommateur en énergie, jusqu'au moment de la première utilisation. Pour "réveiller" l'électronique, il est demandé à l'utilisateur d'actionner le dispositif en inhalant la première dose (si le dispositif est muni d'une valve sans amorçage) ou en réalisant un amorçage (si le dispositif est muni d'une valve classique). Le premier actionnement a pour effet de faire descendre l'organe d'actionnement 800 dans le corps 10. Une partie de l'organe d'actionnement 800 va alors faire pression sur un contacteur 1010, comme représenté sur les figures 2 à 5, ce qui a pour effet de fermer une boucle de courant. Le dispositif détecte alors ce changement d'intensité ce qui va réveiller l'électronique et mettre le compteur en état de fonctionnement normal.

En variante, ou de manière additionnelle, le dispositif peut comporter un accéléromètre.

Cet accéléromètre peut avoir plusieurs fonctions:
- il peut servir à vérifier que le dispositif a bien été secoué avant utilisation ; en effet, la plupart des médicaments conservés dans un réservoir sous pression sont des médicaments plus ou moins solubles et il faut donc les mélanger avant la prise de dose ; si le circuit électrique capte via l'accéléromètre que le dispositif n'a pas été secoué, l'utilisateur pourra en être informé, par exemple par une application sur son smartphone ou sur un écran du dispositif ;
- certaines personnes prennent le dispositif à l'envers lorsqu'ils souhaite l'actionner ; lorsque le dispositif est à l'envers, c'est-à-dire avec le réservoir disposé en-dessous de la valve, le liquide ne peut pas entrer dans la chambre de dosage de la valve, ce qui résulte en une dose réduite voire nulle lors du chargement de la dose ; l'accéléromètre peut détecter si le patient prend le dispositif à l'envers et lui indiquer, de préférence avant la prise de dose, par exemple par un bip sonore et/ou une indication sur l'écran et/ou sur le smartphone, que le dispositif n'est pas dans le bon sens ;
- une autre utilité de l'accéléromètre est de l'utiliser pour économiser de la batterie ; lorsque le dispositif est dans un sac ou une poche de pantalon ou encore posé sur une table, le dispositif est en mode veille ; pendant cette phase, l'écran du compteur et une grande partie de l'électronique embarquée sont en mode veille, ce qui permet de réduire grandement la consommation électrique ; lorsque l'utilisateur se saisit du dispositif, l'accéléromètre détecte le mouvement et réveille l'électronique pour mettre le compteur en état de fonctionnement normal ; cette option permet alors de réduire la capacité de la batterie embarquée et donc d'avoir un impact moins négatif sur l'environnement.

Le dispositif peut aussi comporter des moyens d'émission de signaux pour communiquer à distance notamment des informations relatives aux actionnements du dispositif. En particulier, le corps et/ou le capot et/ou le compteur peut comporter un module d'émission de signaux, pour communiquer à distance avec un dispositif distant quelconque. Des moyens d'alimentation appropriés sont avantageusement prévus.

Avantageusement, le module électronique peut comprendre une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Le commutateur peut être actionné grâce au mouvement de l'organe d'actionnement et/ou de l'élément de blocage et/ou de l'élément déclencheur et/ou de l'organe sensible à l'inhalation.

Associés à un compteur de dose qui compte chaque dose effectivement émise et au dispositif de synchronisation avec l'inhalation de l'invention, ces moyens d'émission de signaux permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution. Le médecin peut ainsi connaitre très exactement les conditions d'utilisation du dispositif par l'utilisateur.

La présente invention est notamment applicable pour le traitement des crises d'asthme ou de BPCO (Broncho Pneumopathie Chronique Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à des modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), un réservoir de produit (100) contenant un produit fluide et un gaz propulseur étant monté axialement coulissant par rapport audit corps (10), une valve doseuse (200), comportant une soupape (210) déplaçable entre une position de repos et une position d'actionnement, étant assemblée sur ledit réservoir (100) pour distribuer sélectivement le produit fluide à chaque actionnement, ledit dispositif comportant:
- un élément de blocage (500) déplaçable et/ou déformable entre une position de blocage, dans laquelle ladite valve doseuse (200) ne peut pas être actionnée, et une position d'actionnement, dans laquelle ladite valve doseuse (200) peut être actionnée,
- un élément déclencheur (600) déplaçable et/ou déformable entre une position de verrouillage, dans laquelle il bloque ledit élément de blocage (500) dans sa position de blocage, et une position de libération, dans laquelle il ne bloque pas ledit élément de blocage (500),
- un système de déclenchement commandé par l'inhalation comportant un organe sensible à l'inhalation (60), déformable et/ou déplaçable sous l'effet de l'inhalation, ledit organe sensible à l'inhalation (60) coopérant avec ledit élément déclencheur (600), de sorte que lorsque ledit organe sensible à l'inhalation (60) se déforme et/ou se déplace, il déplace et/ou déforme ledit élément déclencheur (600) vers sa position de libération, permettant ainsi le déplacement et/ou la déformation dudit élément de blocage (500) de sa position de blocage vers sa position d'actionnement,
- un organe d'actionnement (800), déplaçable entre une position de repos et une position d'actionnement sous l'effet d'un ressort (850), ledit organe d'actionnement (800) coopérant avec ledit élément de blocage (500), de telle sorte qu'en position de blocage dudit élément de blocage (500), ledit élément de blocage (500) empêche un déplacement axial dudit organe d'actionnement (800), et en position d'actionnement dudit élément de blocage (500), ledit élément de blocage (500) permet un déplacement axial dudit organe d'actionnement (800), et
- un compteur de doses (1000), ledit élément de blocage (500) comportant une projection de verrouillage (510) qui, en position de verrouillage de l'élément déclencheur (600), coopère avec un épaulement de verrouillage (610) dudit élément déclencheur (600) pour définir une serrure empêchant le déplacement et/ou la déformation dudit élément de blocage (500) hors de sa position de blocage,
**caractérisé en ce que** ledit dispositif comporte un système de libération automatique de soupape, qui ramène automatiquement ladite soupape (210) de ladite valve (200) vers sa position de repos après actionnement de la valve, indépendamment de la position dudit organe d'actionnement (800).

2. Dispositif selon la revendication 1, dans lequel ledit dispositif comporte un capot (1) déplaçable, notamment pivotant sur la corps (10), entre une position de fermeture de l'embout buccal (400) et une position d'ouverture de l'embout buccal (400), ledit capot (1) coopérant avec ledit organe d'actionnement (800) de telle sorte qu'en position de fermeture, il bloque ledit organe d'actionnement (800) contre un déplacement axial dans le corps (10), et lorsqu'il est ramené de sa position d'ouverture vers sa position de fermeture, il ramène l'organe d'actionnement (800) vers sa position de repos en rechargeant ledit ressort (850).

3. Dispositif selon la revendication 2, dans lequel ledit système de libération automatique de soupape comporte deux leviers (901, 902), un élément de poussée (910) et un ressort de leviers (920), ledit élément de poussée (910) étant destiné à venir en contact avec ledit réservoir (100), ledit élément de poussée (910) étant relié à un organe de poussée (810), avec interposition dudit ressort de leviers (920).

4. Dispositif selon la revendication 3, dans lequel chaque levier (901, 902) est assemblé via des plots (905) de manière pivotante dans une came (815) dudit organe de poussée (810) et coopère avec ledit élément de poussée (910) pour transmettre la force (F) exercée par ledit ressort (850) audit élément de poussée (910).

5. Dispositif selon la revendication 4, dans lequel, en position verrouillée, avant inhalation, lesdits leviers (901, 902) sont d'un côté en appui sur un épaulement (911) dudit élément de poussée (910), et de l'autre côté, lesdits leviers (901, 902) sont en contact de doigts de verrouillage (115) formés dans un couvercle (11) et qui s'étendent axialement vers le bas à partir du fond dudit couvercle (11), lesdits doigts de verrouillage (115) empêchant lesdits leviers (901, 902) de pivoter hors de contact avec ledit épaulement (911) dudit élément de poussée (910).

6. Dispositif selon la revendication 5, dans lequel, en position d'actionnement, après inhalation, lesdits leviers (901, 902) ne coopèrent plus avec lesdits doigts de verrouillage (115) dudit couvercle (11), de sorte qu'ils peuvent alors pivoter dans ledit organe de poussée (810) hors de contact avec ledit épaulement (911) dudit élément de poussée (910), de sorte que ledit réservoir (100) peut remonter vers sa position de repos sous l'effet du ressort de rappel de ladite valve (200), avec lesdits leviers (901, 902) passant à travers des trous (912) dudit élément de poussée (910).

7. Dispositif selon la revendication 6, dans lequel, lors du réarmement du dispositif, lorsque l'utilisateur referme ledit capot (1), ledit organe d'actionnement (800) et ledit organe de poussée (810) reviennent vers leurs positions de repos, en comprimant ledit ressort (850), et lesdits leviers (901, 902) se déplacent axialement avec ledit organe de poussée (810) jusqu'à ce qu'ils entrent en contact avec lesdits doigts de verrouillage (115) dudit couvercle (11), qui sollicitent lesdits plots (905) à se déplacer radialement vers l'extérieur dans lesdits cames (815), ce qui permet auxdits leviers (901, 902) de revenir autour desdits doigts de verrouillage (115), ledit ressort de leviers (920) ramenant alors lesdits leviers (901, 902) en appui sur ledit épaulement (911) dudit élément de poussée (910).

8. Dispositif selon l'une quelconque des revendications précédentes, dans lequel ledit élément de blocage (500) est monté pivotant sur le corps (10) autour d'un axe de pivotement (B) et ledit élément déclencheur (600) est monté pivotant sur le corps (10) autour d'un axe de pivotement (C), lesdits axes (B) et (C) étant parallèles.

9. Dispositif selon l'une quelconque des revendications précédentes, dans lequel ledit organe d'actionnement (800) comporte une projection axiale (801) coopérant avec ledit élément de blocage (500), de telle sorte qu'en position de blocage dudit élément de blocage (500), ladite projection axiale (801) dudit organe d'actionnement (800) coopère avec une extension de blocage axiale (501) dudit élément de blocage (500) pour ainsi empêcher un déplacement axial dudit organe d'actionnement (800), et en position d'actionnement dudit élément de blocage (500), ladite projection axiale (801) dudit organe d'actionnement (800) coopère avec un évidement axial (502) dudit élément de blocage (500) pour ainsi permettre un déplacement axial dudit organe d'actionnement (800).

10. Dispositif selon la revendication 9, dans lequel, en position de blocage dudit élément de blocage (500), ladite projection axiale (801) dudit organe d'actionnement (800) sollicite ledit élément de blocage (500) vers sa position d'actionnement.

11. Dispositif selon l'une quelconque des revendications précédentes, dans lequel ladite serrure forme, en position de verrouillage de l'élément déclencheur (600), un premier point de contact entre ledit élément de blocage (500) et ledit élément déclencheur (600), ledit élément de blocage (500) comportant une projection d'appui (520) qui, en position de verrouillage de l'élément déclencheur (600), coopère avec une surface d'appui (620) dudit élément déclencheur (600) pour former, en position de verrouillage de l'élément déclencheur (600), un second point de contact entre ledit élément de blocage (500) et ledit élément déclencheur (600), ledit second point de contact étant, en position de verrouillage de l'élément déclencheur (600), à une distance dudit axe (C) de l'élément déclencheur (600) supérieure à la distance entre ledit axe (C) et ledit premier point de contact.

12. Dispositif selon l'une quelconque des revendications précédentes, dans lequel ledit dispositif comporte :
- un couvercle (11) fixé, notamment vissé ou encliqueté, sur ledit corps (10), et
- un organe de poussée (810) coopérant avec ledit organe d'actionnement (800) et monté coulissant axialement dans ledit couvercle (11), ledit ressort (850) étant disposé entre un fond dudit couvercle (11) et ledit organe de poussée (810),
dans lequel, avant inhalation, ledit organe de poussée (810) est hors de contact dudit réservoir (100), de sorte que la force exercée sur ledit organe de poussée (810) par ledit ressort (850) n'est pas transmise audit réservoir (100), et lors de l'inhalation, ledit organe de poussée (810) se déplace axialement avec ledit organe d'actionnement (800) pour venir en contact avec ledit réservoir (100) et déplacer ledit réservoir (100) axialement dans ledit corps (10) pour actionner ladite valve (200).

13. Dispositif selon l'une quelconque des revendications précédentes, comportant un indicateur (15) pour indiquer à l'utilisateur que la distribution de produit fluide a été réalisée.

14. Dispositif selon la revendication 13, dans lequel ledit indicateur est un indicateur visuel et/ou sonore.

15. Dispositif selon l'une quelconque des revendications précédentes, dans lequel ledit compteur de doses (1000) est un compteur électronique.

16. Dispositif selon la revendication 15, dans lequel, avant la première utilisation du dispositif, ledit compteur électronique (1000) est en mode veille, ledit compteur électronique comportant un contacteur (1010), ledit organe d'actionnement (800), lors de son premier déplacement vers sa position d'actionnement, contactant ledit contacteur (1010) pour mettre ledit compteur électronique (1000) en mode de fonctionnement normal.

17. Dispositif selon la revendication 15 ou 16, dans lequel ledit compteur électronique (1000) comporte au moins un accéléromètre.

18. Dispositif selon l'une quelconque des revendications précédentes, dans lequel ledit organe sensible à l'inhalation (60) comporte une membrane déformable définissant une chambre d'air déformable, ladite membrane déformable étant fixée audit élément déclencheur (600), ladite membrane déformable étant déformée lors de l'inhalation de telle sorte qu'elle déplace ledit élément déclencheur (600) de sa position de verrouillage vers sa position de libération.

19. Dispositif selon l'une quelconque des revendications précédentes, dans lequel ledit embout buccal (400) comporte une ouverture (410) reliée avec l'intérieur du corps (10), ladite ouverture (410) étant fermée en début d'inhalation par un clapet (420), de sorte que le flux d'inhalation est initialement principalement transmis au système de déclenchement par l'inhalation.

20. Dispositif selon la revendication 19, dans lequel ledit clapet (420) est ouvert lorsque ledit organe d'actionnement (800) se déplace axialement ensemble avec ledit réservoir (100).

## Patentansprüche

1. Vorrichtung zur inhalationssynchronisierten Abgabe eines fluiden Produkts, umfassend einen Körper (10), der mit einem Mundstück (400) versehen ist, wobei ein Produktbehälter (100), der ein fluides Produkt und ein Treibgas enthält, in Bezug auf den Körper (10) axial verschiebbar montiert ist, wobei ein Dosierventil (200), das ein zwischen einer Ruhestellung und einer Betätigungsstellung bewegbares Ventilelement (210) umfasst, am Behälter (100) angebracht ist, um das fluide Produkt bei jeder Betätigung selektiv abzugeben, wobei die Vorrichtung Folgendes umfasst:
- ein Blockierelement (500), das zwischen einer Blockierstellung, in der das Dosierventil (200) nicht betätigt werden kann, und einer Betätigungsstellung, in der das Dosierventil (200) betätigt werden kann, bewegbar und/oder verformbar ist,
- ein Auslöseelement (600), das zwischen einer Verriegelungsstellung, in der es das Blockierelement (500) in seiner Blockierstellung blockiert, und einer Freigabestellung, in der es das Blockierelement (500) nicht blockiert, bewegbar und/oder verformbar ist,
- ein durch die Inhalation gesteuertes Auslösesystem, das ein inhalationsempfindliches Organ (60) umfasst, das unter der Wirkung der Inhalation verformbar und/oder bewegbar ist, wobei das inhalationsempfindliche Organ (60) mit dem Auslöseelement (600) zusammenwirkt, sodass das inhalationsempfindliche Organ (60), wenn es sich verformt und/oder bewegt, das Auslöseelement (600) in seine Freigabestellung bewegt und/oder verformt, wodurch die Bewegung und/oder Verformung des Blockierelements (500) aus seiner Blockierstellung in seine Betätigungsstellung ermöglicht wird,
- ein Betätigungsorgan (800), das unter der Wirkung einer Feder (850) zwischen einer Ruhestellung und einer Betätigungsstellung bewegbar ist, wobei das Betätigungsorgan (800) mit dem Blockierelement (500) zusammenwirkt, sodass das Blockierelement (500) in der Blockierstellung des Blockierelements (500) eine axiale Bewegung des Betätigungsorgans (800) verhindert und das Blockierelement (500) in der Betätigungsstellung des Blockierelements (500) eine axiale Bewegung des Betätigungsorgans (800) zulässt, und
- einen Dosenzähler (1000), wobei das Blockierelement (500) einen Verriegelungsvorsprung (510) umfasst, der in der Verriegelungsstellung des Auslöseelements (600), mit einer Verriegelungsschulter (610) des Auslöseelements (600) zusammenwirkt, um ein Schloss zu definieren, das die Bewegung und/oder die Verformung des Blockierelements (500) aus seiner Blockierstellung heraus verhindert,
**dadurch gekennzeichnet, dass** die Vorrichtung ein automatisches Ventilfreigabesystem umfasst, das das Ventilelement (210) des Ventils (200) nach der Betätigung des Ventils unabhängig von der Stellung des Betätigungsorgans (800) automatisch in seine Ruhestellung zurückführt.

2. Vorrichtung nach Anspruch 1, wobei die Vorrichtung eine Abdeckung (1) umfasst, die zwischen einer Schließstellung des Mundstücks (400) und einer Öffnungsstellung des Mundstücks (400) bewegbar ist, insbesondere am Körper (10) schwenkbar, wobei die Abdeckung (1) mit dem Betätigungsorgan (800) so zusammenwirkt, dass sie in der Schließstellung das Betätigungsorgan (800) gegen eine axiale Bewegung im Körper (10) blockiert und das sie, wenn sie aus ihrer Öffnungsstellung in ihre Schließstellung zurückgeführt wird, das Betätigungsorgan (800) in seine Ruhestellung zurückführt und dabei die Feder (850) wieder belastet.

3. Vorrichtung nach Anspruch 2, wobei das automatische Ventilfreigabesystemelement zwei Hebel (901, 902), ein Schubelement (910) und eine Hebelfeder (920) umfasst, wobei das Schubelement (910) dazu bestimmt ist, mit dem Behälter (100) in Kontakt zu kommen, wobei das Schubelement (910) unter Zwischenschaltung der Hebelfeder (920) mit einem Schuborgan (810) verbunden ist.

4. Vorrichtung nach Anspruch 3, wobei jeder Hebel (901, 902) über Noppen (905) schwenkbar in einem Nocken (815) des Schuborgans (810) angebracht ist und mit dem Schubelement (910) zusammenwirkt, um die durch die Feder (850) ausgeübte Kraft (F) auf das Schubelement (910) zu übertragen.

5. Vorrichtung nach Anspruch 4, wobei die Hebel (901, 902) in der Verriegelungsstellung vor der Inhalation auf einer Seite an einer Schulter (911) des Schubelements (910) anliegen und wobei die Hebel (901, 902) auf der anderen Seite mit Verriegelungsfingern (115) in Kontakt stehen, die in einem Deckel (11) ausgebildet sind und die sich vom Boden des Deckels (11) axial nach unten erstrecken, wobei die Verriegelungsfinger (115) verhindern, dass sich die Hebel (901, 902) aus dem Kontakt mit der Schulter (911) des Schubelements (910) herausschwenken.

6. Vorrichtung nach Anspruch 5, wobei die Hebel (901, 902) in der Betätigungsstellung nach der Inhalation nicht mehr mit den Verriegelungsfingern (115) des Deckels (11) zusammenwirken, sodass sie dann im Schuborgan (810) aus dem Kontakt mit der Schulter (911) des Schubelements (910) herausschwenken können, sodass der Behälter (100) unter der Wirkung der Rückstellfeder des Ventils (200) in seine Ruhestellung zurückkehren kann, wobei die Hebel (901, 902) durch Löcher (912) des Schubelements (910) hindurchlaufen.

7. Vorrichtung nach Anspruch 6, wobei das Betätigungsorgan (800) und das Schuborgan (810) beim Zurücksetzen der Vorrichtung, wenn der Benutzer die Abdeckung (1) wieder schließt, in ihre Ruhestellungen zurückkehren, wobei die Feder (850) zusammengedrückt wird und die Hebel (901, 902) sich zusammen mit dem Schuborgan (810) axial bewegen, bis sie mit den Verriegelungsfingern (115) des Deckels (11) in Kontakt kommen, die die Noppen (905) dazu drängen, sich in den Nocken (815) radial nach außen zu bewegen, wodurch die Hebel (901, 902) um die Verriegelungsfinger (115) herum zurückkehren können, wobei die Hebelfeder (920) dann die Hebel (901, 902) in Anlage an der Schulter (911) des Schubelements (910) zurückbringt.

8. Vorrichtung nach einem der vorangehenden Ansprüche, wobei das Blockierelement (500) um eine Schwenkachse (B) schwenkbar am Körper (10) montiert ist und das Auslöseelement (600) um eine Schwenkachse (C) schwenkbar am Körper (10) montiert ist, wobei die Achsen (B) und (C) parallel sind.

9. Vorrichtung nach einem der vorangehenden Ansprüche, wobei das Betätigungsorgan (800) einen axialen Vorsprung (801) umfasst, der mit dem Blockierelement (500) zusammenwirkt, so dass der axiale Vorsprung (801) des Betätigungsorgans (800) in der Blockierstellung des Blockierelements (500) mit einer axialen Blockiererstreckung (501) des Blockierelements (500) zusammenwirkt, um so eine axiale Bewegung des Betätigungsorgans (800) zu verhindern, und wobei der axiale Vorsprung (801) des Betätigungsorgans (800) in der Betätigungsstellung des Blockierelements (500) mit einer axialen Aussparung (502) des Blockierelements (500) zusammenwirkt, um so eine axiale Bewegung des Betätigungsorgans (800) zuzulassen.

10. Vorrichtung nach Anspruch 9, wobei der axiale Vorsprung (801) des Betätigungsorgans (800) in der Blockierstellung des Blockierelements (500) das Blockierelement (500) in seine Betätigungsstellung drängt.

11. Vorrichtung nach einem der vorangehenden Ansprüche, wobei das Schloss in der Verriegelungsstellung des Auslöseelements (600) einen ersten Kontaktpunkt zwischen dem Blockierelement (500) und dem Auslöseelement (600) ausbildet, wobei das Blockierelement (500) einen Auflagevorsprung (520) umfasst, der in der Verriegelungsstellung des Auslöseelements (600) mit einer Auflagefläche (620) des Auslöseelements (600) zusammenwirkt, um in der Verriegelungsstellung des Auslöseelements (600), einen zweiten Kontaktpunkt zwischen dem Blockierelement (500) und dem Auslöseelement (600) auszubilden, wobei sich der zweite Kontaktpunkt in der Verriegelungsstellung des Auslöseelements (600) in einem Abstand zur Achse (C) des Auslöseelements (600) befindet, der größer als der Abstand zwischen der Achse (C) und dem ersten Kontaktpunkt ist.

12. Vorrichtung nach einem der vorangehenden Ansprüche, wobei die Vorrichtung Folgendes umfasst:
- einen Deckel (11), der auf dem Körper (10) befestigt, insbesondere aufgeschraubt oder aufgerastet ist, und
- ein Schuborgan (810), das mit dem Betätigungsorgan (800) zusammenwirkt und axial verschiebbar im Deckel (11) montiert ist, wobei die Feder (850) zwischen einem Boden des Deckels (11) und dem Schuborgan (810) angeordnet ist,
wobei das Schuborgan (810) vor der Inhalation außer Kontakt mit dem Behälter (100) steht, sodass die durch die Feder (850) auf das Schuborgan (810) ausgeübte Kraft nicht auf den Behälter (100) übertragen wird, und wobei sich das Schuborgan (810) während der Inhalation zusammen mit dem Betätigungsorgan (800) axial bewegt, um mit dem Behälter (100) in Kontakt zu kommen und den Behälter (100) axial im Körper (10) zu bewegen, um das Ventil (200) zu betätigen.

13. Vorrichtung nach einem der vorangehenden Ansprüche, umfassend einen Indikator (15), um dem Benutzer anzuzeigen, dass die Abgabe eines fluiden Produkts erfolgt ist.

14. Vorrichtung nach Anspruch 13, wobei es sich bei dem Indikator um einen visuellen und/oder akustischen Indikator handelt.

15. Vorrichtung nach einem der vorangehenden Ansprüche, wobei es sich bei dem Dosenzähler (1000) um einen elektronischen Zähler handelt.

16. Vorrichtung nach Anspruch 15, wobei sich der elektronische Zähler (1000) vor der ersten Verwendung der Vorrichtung im Bereitschaftsmodus befindet, wobei der elektronische Zähler einen Kontaktgeber (1010) umfasst, wobei das Betätigungsorgan (800) bei seiner ersten Bewegung in seine Betätigungsstellung mit dem Kontaktgeber (1010) in Kontakt kommt, um den elektronischen Zähler (1000) in den Normalbetriebsmodus zu versetzen.

17. Vorrichtung nach Anspruch 15 oder 16, wobei der elektronische Zähler (1000) mindestens einen Beschleunigungsmesser umfasst.

18. Vorrichtung nach einem der vorangehenden Ansprüche, wobei das inhalationsempfindliche Organ (60) eine verformbare Membran umfasst, die eine verformbare Luftkammer definiert, wobei die verformbare Membran am Auslöseelement (600) befestigt ist, wobei die verformbare Membran während der Inhalation so verformt wird, dass sie das Auslöseelement (600) aus seiner Verriegelungsstellung in seine Freigabestellung bewegt.

19. Vorrichtung nach einem der vorangehenden Ansprüche, wobei das Mundstück (400) eine Öffnung (410) umfasst, die mit dem Inneren des Körpers (10) verbunden ist, wobei die Öffnung (410) zu Beginn der Inhalation durch eine Klappe (420) verschlossen ist, sodass der Inhalationsstrom anfangs hauptsächlich an das Inhalationsauslösesystem weitergeleitet wird.

20. Vorrichtung nach Anspruch 19, wobei die Klappe (420) offen ist, wenn sich das Betätigungsorgan (800) zusammen mit dem Behälter (100) axial bewegt.

## Claims

1. An inhalation-synchronized fluid product dispenser device comprising a body (10) provided with a mouthpiece (400), a product reservoir (100) containing a fluid product and a propellant gas being mounted to slide axially relative to said body (10), a metering valve (200), comprising a valve member (210) movable between a rest position and an actuation position, being assembled on said reservoir (100) for selectively dispensing the fluid product on each actuation, said device further comprising:
- a blocking element (500) that is movable and/or deformable between a blocking position in which said metering valve (200) cannot be actuated, and an actuation position in which said metering valve (200), can be actuated,
- a trigger element (600) that is movable and/or deformable between a locking position in which it blocks said blocking element (500) in its blocking position, and a release position wherein it does not block said blocking element (500),
- an inhalation-controlled trigger system comprising an inhalation-sensitive member (60) that is deformable and/or movable under the effect of inhaling, said inhalation-sensitive member (60) cooperating with said trigger element (600), such that when said inhalation-sensitive member (60) is deformed and/or moved, it moves and/or deforms said trigger element (600) towards its release position, thus making it possible to move and/or deform said blocking element (500) from its blocking position towards its actuation position,
- an actuating member (800) that is movable between a rest position and an actuation position under the effect of a spring (850), said actuating member (800) cooperating with said blocking element (500) such that in blocking position said blocking element (500), said blocking element (500) prevents an axial movement of said actuating member (800), and in actuation position said blocking element (500), said blocking element (500) makes it possible to move said actuating member (800) axially, and
- a dose meter (1000), said blocking element (500) comprising a locking projection (510) that, in the locking position of the trigger element (600), cooperates with a locking shoulder (610) of said trigger element (600) so as to define a latch that prevents said blocking element (500) from moving and/or deforming out of its blocking position, **characterized in that** said device comprising an automatic valve release system, which automatically returns said valve member (210) from said valve (200) towards its rest position after actuation of the valve, independently of the position of said actuating member (800).

2. The device according to claim 1, wherein said device comprises a cap (1) which can be moved, in particular pivoting on the body (10), between a closed position of the mouthpiece (400) and an open position of the mouthpiece (400), said cap (1) cooperating with the said actuating member (800) such that, in the closed position, it blocks said actuating member (800) against an axial movement in the body (10), and when it is returned from its open position to its closed position, it returns the actuating member (800) to its rest position by reloading said spring (850).

3. The device according to claim 2, wherein said automatic valve release system comprises two levers (901, 902), a push element (910) and a lever spring (920), said push element (910) being intended to come into contact with said reservoir (100), said push element (910) being connected to a push member (810), with the interposition of said lever spring (920).

4. The device according to claim 3, wherein each lever (901, 902) is assembled via studs (905) so as to pivot in a cam (815) of said push member (810) and cooperates with said push element (910) so as to transmit the force (F) exerted by said spring (850) to said push element (910).

5. The device according to claim 4, wherein in the locked position, before inhalation, said levers (901, 902) bear on one side on a shoulder (911) of said push element (910), and on the other side said levers (901, 902) are in contact with locking fingers (115) formed in a cover (11) and which extend axially downwards from the bottom of said cover (11), said locking fingers (115) preventing said levers (901, 902) from pivoting out of contact with said shoulder (911) of said push element (910).

6. The device according to claim 5, wherein, in the actuation position, after inhalation said levers (901, 902) no longer cooperate with said locking fingers (115) of said cover (11), such that they can then pivot in said push member (810) out of contact with said shoulder (911) of said push element (910), such that said reservoir (100) can rise towards its rest position under the effect of the return spring of said valve (200), with said levers (901, 902) passing through holes (912) in said push element (910).

7. The device according to claim 6, wherein when the device is reset, when the user closes said cap (1), said actuating member (800) and said push member (810) return to their rest positions, compressing said spring (850), and said levers (901, 902) move axially with said push member (810) until they come into contact with said locking fingers (115) of said cap (11), which urge said studs (905) to move radially outwards in said cams (815), which allows said levers (901, 902) to return around said locking fingers (115), said lever spring (920) then returning said levers (901, 902) to bear on said shoulder (911) of said push member (910).

8. The device according to any one of preceding claims, wherein said blocking element (500) is mounted to pivot on the body (10) about a pivot axis B, and said trigger element (600) is mounted to pivot on the body (10) about a pivot axis (C), said axes (B) and (C) being parallel.

9. The device according to any one of the preceding claims, wherein said actuating member (800) comprises an axial projection (801) cooperating with said blocking element (500), such that in the blocking position of said blocking element (500), said axial projection (801) of said actuating member (800) cooperates with an axial blocking extension (501) of said blocking element (500) to thus prevent an axial movement of said actuating member (800), and in the actuation position of said blocking element (500), said axial projection (801) of said actuating member (800) cooperates with an axial recess (502) of said blocking element (500) thereby enabling said reservoir (800) to move axially.

10. The device according to claim 9, wherein, in the blocking position of said blocking element (500), said axial projection (801) of said actuating member (800) urges said blocking element (500) towards its actuation position.

11. The device according to any one of the preceding claims, wherein in the locking position of the trigger element (600), said latch forms a first contact point between said blocking element (500) and said trigger element (600), said blocking element (500) comprising a bearing projection (520) that, in the locking position of the trigger element (600), cooperates with a bearing surface (620) of said trigger element (600) so as to form, in the locking position of the trigger element (600), a second contact point between said blocking element (500) and said trigger element (600), said second contact point being, in the locking position of the trigger element (600), at a distance from said axis (C) of the trigger element (600) that is greater than the distance between said axis (C) and said first contact point.

12. The device according to any one of preceding claims, wherein said device comprises:
- a cover (11) which is fixed, in particular screwed or snap-fitted, to said body (10), and
- a push member (810) cooperating with said actuating member (800) and mounted to slide axially in said cover (11), said spring (850) being disposed between a bottom of said cover (11) and said push member (810),
wherein, before inhalation, said push member (810) is out of contact with said reservoir (100), such that the force exerted on said push member (810) by said spring (850) is not transmitted to said reservoir (100), and during inhalation, said push member (810) moves axially with said actuating member (800) so as to come into contact with said reservoir (100) and move said reservoir (100) axially in said body (10) so as to actuate said valve (200).

13. The device according to any one of the preceding claims, comprising an indicator (15) for indicating to the user that the dispensing of fluid product has been made.

14. The device according to claim 13, wherein said indicator is a visual indicator and/or audible indicator.

15. The device according to any one of the preceding claims, wherein said dose meter (1000) is an electronic meter (1000).

16. The device according to claim 15, wherein, before the first use of the device, said electronic meter (1000) is in standby mode, said electronic counter comprising a contactor (1010), said actuating member (800), during its first movement towards its actuation position, contacting said contactor (1010) so as to place said electronic meter (1000) in normal operating mode.

17. The device according to claim 15 or 16, wherein said electronic meter (1000) comprises at least one accelerometer.

18. The device according to any one of the preceding claims, wherein said inhalation-sensitive member (60) comprises a deformable membrane that defines a deformable air chamber, said deformable membrane being fixed to said trigger element (600), said deformable membrane being deformed during inhaling, such that it moves said trigger element (600) from its locking position towards its release position.

19. The device according to any one of the preceding claims, wherein said mouthpiece (400) comprises an opening (410) that is connected to the inside of the body (10), said opening (410) being closed at the start of inhaling by a check valve (420), such that the inhalation flow due to inhaling initially passes mainly to the trigger system.

20. The device according to claim 19, wherein said check valve (420) is opened when said actuating member (800) moves axially together with said reservoir (100).
